# FASCICULE DE BREVET EUROPEEN

(11) **EP 3 429 511 B1**
(45) Date de publication et mention de la délivrance du brevet: **13.11.2019**
(21) Numéro de dépôt: 17714863.2
(22) Date de dépôt: 15.03.2017
(51) Int. Cl.: A61F 2/42, A61F 2/46, A61B 17/72, A61B 17/88, A61F 2/30

(54) **IMPLANT MÉDICAL ET ENSEMBLE COMPRENANT UN TEL IMPLANT MÉDICAL ET UN PRÉHENSEUR DUDIT IMPLANT**
MEDIZINISCHES IMPLANTAT UND ENSEMBLE BESTEHEND AUS EIN SOLCHES IMPLANTAT UND EIN GREIFER FÜR BESAGTES IMPLANTAT
MEDICAL IMPLANT AND ASSEMBLY COMPRISING SUCH AN IMPLANT AND A GRIPPER FOR SAID IMPLANT

(30) Priorité: 16.03.2016 FR 1652221
(43) Date de publication de la demande: 23.01.2019
(73) Titulaire: Neosteo, 44100 Nantes (FR)
(72) Inventeur: DEROUET, Guillaume, 44700 Orvault (FR); DECHELETTE, Maxime, 44390 Petit Mars (FR); SORIN, Sylvain, 85530 La Bruffiere (FR)
(74) Mandataire: Godineau, Valérie
(86) Numéro de dépôt international: PCT/FR2017/050589
(87) Numéro de publication internationale: WO 2017/158289

(56) Documents cités:
- EP-A1- 1 923 012
- EP-A2- 2 749 236
- FR-A1- 2 787 313
- US-A1- 2015 073 413
- US-A1- 2015 141 994

## Description

### DOMAINE DE L'INVENTION

L'invention concerne un implant médical et un ensemble comprenant un tel implant médical et un préhenseur dudit implant.

Elle concerne plus particulièrement un implant médical destiné à permettre la fusion osseuse entre un premier os et un deuxième os, ledit implant se présentant sous forme d'une pièce allongée comprenant, s'étendant suivant l'axe longitudinal de la pièce, une tête et un corps séparés l'un de l'autre par une zone de liaison de la tête au corps, ladite tête étant apte à être insérée dans le premier os, le corps étant apte à être introduit dans le deuxième os, ledit corps étant un corps fendu longitudinalement sur au moins une partie de sa longueur pour délimiter au moins trois pattes longitudinales élastiquement déformables par lesquelles ledit corps est apte à être introduit dans le deuxième os.

### ART ANTÉRIEUR

Un implant médical destiné à permettre le maintien à l'état accolé de deux os par aboutement pendant le temps nécessaire à la fusion des os accolés est connu de l'état de la technique, comme l'illustrent par exemple les brevets européens EP 2 544 633 et FR 2 913 876.

Un tel implant médical est notamment utilisé lors de la pratique d'une arthrodèse interphalangienne, en particulier au niveau des doigts et des orteils, pour bloquer par voie chirurgicale l'articulation entre deux phalanges.

Pour un résultat optimal, il est nécessaire que l'implant s'adapte au mieux à son environnement et soit positionné de la manière la plus stable possible, pour permettre une consolidation des os dans la position choisie par le chirurgien.

Des implants intramédullaires, tels que celui décrit dans le document US 2015/0141994, ne conviennent pas car le corps n'est pas creux de sorte que la reconstitution osseuse ne peut pas s'opérer. Il en résulte également que les pattes ne sont pas, dans ce cas, délimitées par des fentes longitudinales du corps creux.

De même, dans le document US 2015/0073413, la face externe des pattes en escalier empêche toute introduction aisée du corps de l'implant dans un os.

### BUT ET RÉSUMÉ

Un but de l'invention est de proposer un implant médical du type précité, dont la conception permet une adaptation optimale à son environnement et une stabilité accrue.

Un autre but de l'invention est de proposer un implant médical du type précité, dont la conception permet une pose facilitée.

À cet effet, l'invention a pour objet un implant médical destiné à permettre la fusion osseuse entre un premier os et un deuxième os, ledit implant se présentant sous forme d'une pièce allongée comprenant, s'étendant suivant l'axe longitudinal de la pièce, une tête et un corps séparés l'un de l'autre par une zone de liaison de la tête au corps, ladite tête étant apte à être insérée dans le premier os, ledit corps étant apte à être introduit dans le deuxième os, ledit corps étant un corps creux fendu longitudinalement sur au moins une partie de sa longueur pour délimiter au moins trois pattes longitudinales avec chaque patte longitudinale séparée d'une autre patte longitudinale par une fente longitudinale, lesdites pattes longitudinales par lesquelles ledit corps est apte à être introduit dans le deuxième os étant des pattes élastiquement déformables, caractérisé en ce que chaque patte longitudinale du corps est une patte arquée qui présente une face externe, c'est-à-dire tournée vers l'extérieur du corps, se développant longitudinalement de manière courbe avec une courbure à concavité tournée vers l'extérieur du corps et en ce que chaque patte arquée présente, dans sa partie en forme d'arc, une section transversale sensiblement constante.

La réalisation du corps de l'implant sous forme d'un corps creux permet de faciliter la reconstitution osseuse. Chaque patte longitudinale est séparée d'une autre patte longitudinale par une fente longitudinale. Chaque patte longitudinale est donc délimitée par des fentes longitudinales. Lesdites pattes longitudinales délimitent collectivement la structure circonférentielle du corps au niveau desdites pattes. Les pattes longitudinales sont raccordées au reste du corps sensiblement à un même niveau pris suivant la direction longitudinale. En d'autres termes, les pattes ne sont pas décalées axialement le long dudit corps. Ainsi, les extrémités libres des pattes longitudinales sont disposées sensiblement dans un même plan perpendiculaire à l'axe longitudinal du corps et les zones de raccordement des pattes au reste du corps sont, de manière similaire, disposées dans un même plan perpendiculaire à l'axe longitudinal du corps à l'état non sollicité de l'implant.

La forme des faces externes des pattes arquées permet une adaptation optimale au fût médullaire. En effet, les pattes sont aptes à épouser la forme du profil interne du fût médullaire et une meilleure résistance à l'arrachement est obtenue. La flexibilité et la forme des pattes permettent ainsi, par exemple, un passage facilité du goulot créé par les corticales au centre de la diaphyse de l'os.

Selon un mode de réalisation, l'implant est une tête plate avec deux faces opposées parallèles entre elles ou convergentes en direction du sommet de la tête. Il en résulte un encombrement réduit de la tête.

Selon un mode de réalisation, l'implant est une tête de forme générale triangulaire en pointe de flèche. Cette forme de la tête permet un impact direct dans la phalange distale, sans préparation préalable. Il en résulte une pose.

facilitée.

Selon un mode de réalisation, les côtés du triangle reliant le sommet à la base du triangle sont crantés à la manière de crans de sapin. Ces crans assurent un meilleur maintien de l'implant dans l'os.

Selon un mode de réalisation, la tête et la zone de liaison de la tête au corps de l'implant sont évidées centralement pour délimiter un passage axial traversant débouchant à l'intérieur du corps. Cet espace de reconstruction central et entre les pattes permet d'améliorer la stabilité primaire et d'améliorer la qualité de l'ostéogenèse naturelle, y compris dans l'axe du canal médullaire.

Selon un mode de réalisation, l'implant comprend, au niveau de la zone de liaison de la tête au corps, au moins deux encoches aptes à permettre la saisie de l'implant à l'aide d'un préhenseur. La présence de ces encoches permet un positionnement du préhenseur perpendiculairement à l'axe longitudinal de la pièce constitutive de l'implant. Le préhenseur empêche le recul de l'implant et forme une surface d'appui de la phalange distale lors de son aboutement.

Selon un mode de réalisation, chaque encoche est disposée à la base d'un côté du triangle. Les encoches sont ainsi circonférentiellement opposées, pour faciliter la prise de l'implant.

Selon un mode de réalisation, les faces externes des pattes sont munies au niveau des extrémités libres des pattes et de la zone de raccordement des pattes au reste du corps de nervures ou crans s'étendant transversalement à l'axe longitudinal des pattes. Ces nervures ou crans augmentent la stabilité de l'implant à l'intérieur de l'os.

Selon un mode de réalisation, les extrémités libres des pattes sont à bout arrondi. Il en résulte une facilité d'introduction des pattes dans l'os.

Selon un mode de réalisation, l'arc formé au niveau de chaque patte arquée entre l'extrémité libre de la patte et la zone de raccordement de ladite patte au reste du corps s'étend sur au moins 80% de la longueur de la patte.

Chaque patte arquée présente, dans sa partie en forme d'arc, une section transversale sensiblement constante. Par sensiblement constante, on entend une section transversale constante à ± 10% près.

Selon un mode de réalisation, chaque patte arquée, dans sa partie en forme d'arc, se développe depuis l'extrémité libre de la patte vers sa zone de raccordement au reste du corps, en se rapprochant de l'axe longitudinal du corps puis en s'écartant de l'axe longitudinal dudit corps, la zone de transition entre zone de rapprochement et zone d'écartement étant disposée entre l'extrémité libre de la patte et les 2/3 de la longueur de la patte prise à partir de l'extrémité libre de la patte, de préférence entre le premier tiers et le deuxième tiers de la de la longueur de la patte prise à partir de l'extrémité libre de la patte.

Selon un mode de réalisation, D1 correspondant au diamètre du cercle le plus petit à l'intérieur duquel les faces externes des pattes s'inscrivent, D2 au diamètre du cercle le plus petit à l'intérieur duquel les faces internes des pattes s'inscrivent et D3 à la distance minimale entre deux pattes adjacentes, : D2 ≥ (D1 - D2)/2 et , de préférence, D3 ≥ (D1 - D2)/2.
L'invention a encore pour objet un ensemble du type comprenant un implant médical destiné à permettre la fusion osseuse entre un premier os et un deuxième os, et un préhenseur, ledit implant se présentant sous forme d'une pièce allongée comprenant, s'étendant suivant l'axe longitudinal de la pièce, une tête et un corps séparés l'un de l'autre par une zone de liaison de la tête au corps, ladite tête étant apte à être insérée dans le premier os, ledit corps étant apte à être introduit dans le deuxième os, ledit corps étant un corps creux fendu longitudinalement sur au moins une partie de sa longueur pour délimiter au moins trois pattes longitudinales avec chaque patte longitudinale séparée d'une autre patte longitudinale par une fente longitudinale, lesdites pattes longitudinales par lesquelles ledit corps est apte à être introduit dans le deuxième os étant des pattes élastiquement déformables, caractérisé en ce que l'implant médical est du type précité.

L'invention peut être utilisée dans un procédé de pose d'un implant médical destiné à permettre la fusion osseuse entre un premier os et un deuxième os, à l'aide d'un préhenseur, ledit implant se présentant sous forme d'une pièce allongée comprenant, s'étendant suivant l'axe longitudinal de la pièce, une tête et un corps séparés l'un de l'autre par une zone de liaison de la tête au corps, ledit corps étant un corps creux fendu longitudinalement sur au moins une partie de sa longueur pour délimiter au moins trois pattes longitudinales avec chaque patte longitudinale séparée d'une autre patte longitudinale par une fente longitudinale, lesdites pattes longitudinales, par lesquelles ledit corps est apte à être introduit dans le deuxième os, étant des pattes élastiquement déformables, caractérisé en ce que, l'implant étant saisi par le préhenseur au niveau de sa zone de liaison, et chaque patte longitudinale étant une patte arquée présentant une face externe, c'est-à-dire tournée vers l'extérieur du corps se développant longitudinalement de manière courbe avec une courbure à concavité tournée vers l'extérieur du corps, le procédé comprend une étape d'insertion à l'aide du préhenseur, du corps de l'implant dans un os, appelé deuxième os, tel qu'une phalange proximale, jusqu'à positionnement du préhenseur contre l'extrémité dudit os et une étape d'insertion de la tête de l'implant dans un os, appelé premier os, par aboutement dudit premier os contre le deuxième os, jusqu'à positionnement du premier os en appui contre le préhenseur, une étape d'enlèvement du préhenseur et une étape de terminaison de l'aboutement pour combler l'espace préalablement occupé par le préhenseur.

### DESCRIPTION DÉTAILLÉE DES FIGURES

L'invention sera bien comprise à la lecture de la description suivante d'exemples de réalisation, en référence aux dessins annexés dans lesquels :

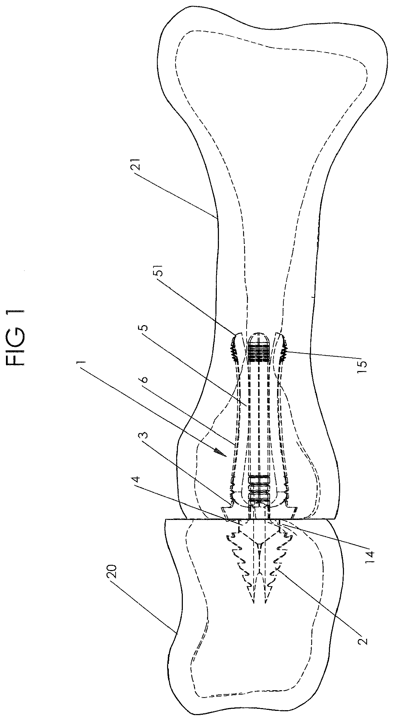

- La figure 1 représente une vue en transparence d'un premier et d'un deuxième os à l'état monté d'un implant médical conforme à l'invention.

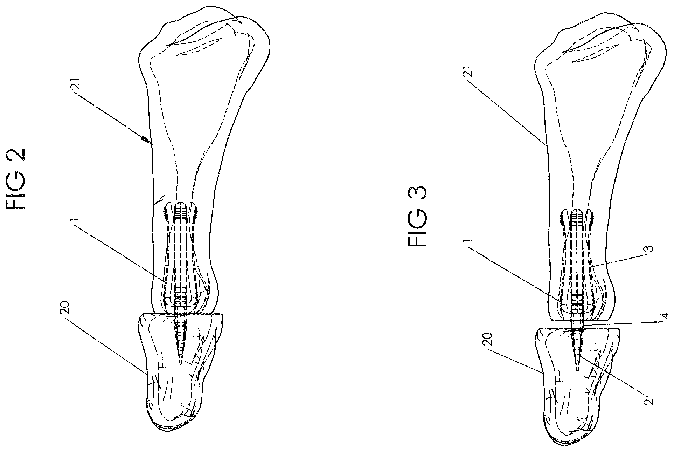

- La figure 2 représente, prise sous un autre angle, une vue en transparence d'un premier et d'un deuxième os à l'état monté d'un implant médical conforme à l'invention.
- La figure 3 représente une vue en transparence d'un premier et d'un deuxième os, à l'état monté d'un implant médical conforme à l'invention, l'aboutement étant en cours d'achèvement, le préhenseur venant d'être enlevé.
- La figure 4 représente une vue en perspective d'un implant médical conforme à l'invention.
- La figure 5 représente une vue en perspective, prise sous un autre angle, d'un implant médical conforme à l'invention.
- La figure 6 représente une vue en perspective, prise sous un autre angle, d'un implant médical conforme à l'invention et une vue de face prise côté sommet de la tête de l'implant.
- La figure 7 représente une vue en perspective d'un implant médical conforme à l'invention, associée à une vue en coupe suivant AA dudit implant.
- La figure 8 représente une vue en coupe longitudinale d'un implant médical conforme à l'invention.
- La figure 9 représente une vue du préhenseur seul et une vue du préhenseur à l'état monté sur un implant.

Comme mentionné ci-dessus, l'invention a pour objet un implant 1 médical destiné à permettre la fusion osseuse entre un premier os 20 formé ici par une phalange distale, et un deuxième os 21 formé par une phalange proximale. L'implant 1 est donc destiné ici à permettre la réalisation d'une arthrodèse interphalangienne.

Cet implant 1 se présente sous forme d'une pièce allongée réalisée monobloc. Cet implant peut être réalisé, par exemple, par injection, par moulage ou encore par usinage. Le matériau convenant pour la réalisation d'un tel implant 1 peut être tout matériau biocompatible, métallique ou non. Par exemple, ce matériau peut être le titane.

Cet implant 1 comprend, s'étendant suivant l'axe longitudinal de la pièce, une tête 2 et un corps 3 séparés l'un de l'autre par une zone 4 de liaison de la tête 2 au corps 3.

La tête 2 est destinée à être insérée dans le premier os 20, tandis que le corps 2 est destiné à être introduit dans le deuxième os 21. Bien évidemment, une solution inverse aurait pu être envisagée, même si elle n'est pas préférée.

Le corps 3 est un corps creux fendu longitudinalement sur une partie de sa longueur pour délimiter ici quatre pattes 5 longitudinales. Chaque patte longitudinale est séparée d'une autre patte longitudinale par une fente longitudinale. Chaque patte longitudinale est donc délimitée par des fentes longitudinales. Lesdites pattes longitudinales délimitent collectivement la structure circonférentielle du corps au niveau desdites pattes. Les pattes longitudinales sont raccordées au reste du corps sensiblement à un même niveau pris suivant la direction longitudinale. En d'autres termes, les pattes ne sont pas décalées axialement le long dudit corps. Ainsi, les extrémités libres des pattes longitudinales sont disposées sensiblement dans un même plan perpendiculaire à l'axe longitudinal du corps et les zones de raccordement des pattes au reste du corps sont, de manière similaire, disposées dans un même plan perpendiculaire à l'axe longitudinal du corps. Le nombre de fentes longitudinales du corps est égal au nombre de pattes 5 longitudinales du corps 3. Ces pattes 5 longitudinales sont des pattes arquées, c'est-à-dire en forme générale d'arc. En particulier, ces pattes 5 longitudinales présentent une face 6 externe, c'est-à-dire tournée vers l'extérieur du corps 3 se développant longitudinalement de manière courbe, avec une courbure à concavité tournée vers l'extérieur du corps 3. Cette courbure suit une ligne courbe en forme d'arc de cercle par opposition à une ligne brisée.

Chaque patte 5 longitudinale élastiquement déformable présente également une face 7 interne, c'est-à-dire tournée vers l'intérieur du corps et opposée à la face 6 externe, se développant également longitudinalement de manière courbe, avec une convexité tournée vers l'intérieur du corps. Il en résulte une finesse des pattes 5 longitudinales courbes, qui permet de faciliter leur introduction dans l'os, sans nuire à leur stabilité dans l'os.

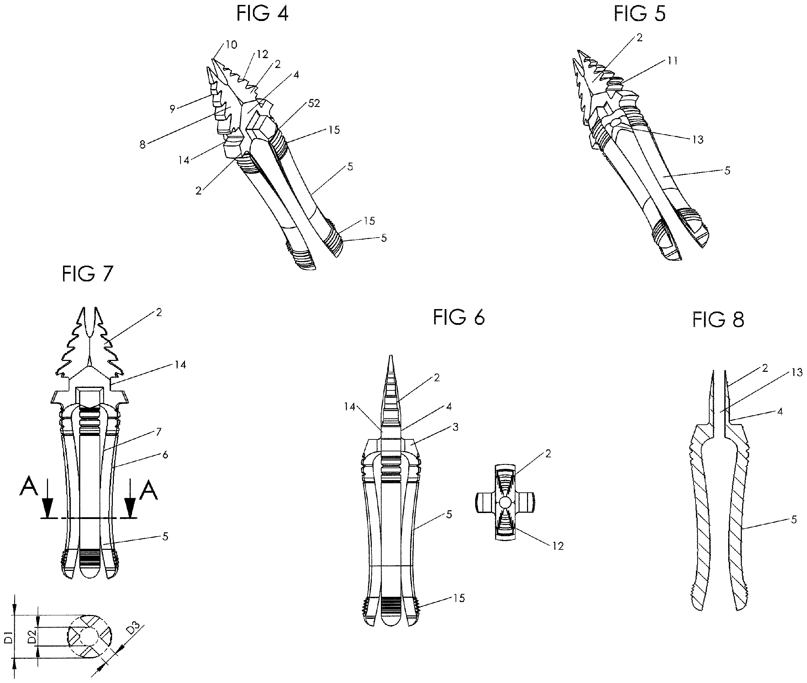

Chaque patte 5 arquée présente, dans sa partie en forme d'arc, une section transversale sensiblement constante. Ainsi, chaque patte 5 arquée présente une section transversale sensiblement constante sur au moins 80 % de sa longueur. Ainsi, chaque patte 5 arquée présente une section transversale sensiblement constante sur au moins 80 % de sa longueur. L'arc formé au niveau de chaque patte 5 arquée entre l'extrémité 51 libre de la patte 5 et la zone 52 de raccordement de ladite patte 5 au reste du corps 3 s'étend sur au moins 80% de la longueur de la patte 5. Ainsi, chaque patte 5 arquée, dans sa partie en forme d'arc, se développe depuis l'extrémité 51 libre de la patte 5 vers sa zone 52 de raccordement au reste du corps 3, en se rapprochant de l'axe longitudinal du corps 3 puis en s'écartant de l'axe longitudinal dudit corps 3, la zone de transition entre zone de rapprochement et zone d'écartement étant disposée entre l'extrémité 51 libre de la patte 5 et les 2/3 de la longueur de la patte prise à partir de l'extrémité 51 libre de la patte 5, de préférence entre le premier tiers et le deuxième tiers de la de la longueur de la patte 5 prise à partir de l'extrémité 51 libre de la patte 5.
Dans l'exemple représenté à la figure 7, D1 correspondant au diamètre du cercle le plus petit à l'intérieur duquel les faces 6 externes des pattes s'inscrivent, D2 au diamètre du cercle le plus petit à l'intérieur duquel les faces 7 internes des pattes s'inscrivent et D3 à la distance minimale entre deux pattes (5) adjacentes,
: D2 ≥ (D1 - D2)/2 et , de préférence, D3 ≥ (D1 - D2)/2.

Les faces 6 externes des pattes 5 sont munies, au niveau des extrémités 51 libres des pattes 5 et de la zone 52 de raccordement des pattes 5 au reste du corps 3, de nervures 15 ou crans s'étendant transversalement à l'axe longitudinal des pattes 5. Ces nervures sont ici des nervures parallèles entre elles et sensiblement perpendiculaires à l'axe longitudinal de la pièce.

Les extrémités libres des pattes sont à bout arrondi. À nouveau, cette disposition permet de faciliter l'introduction des pattes dans l'os associé.

La tête 2 de l'implant 1 est quant à elle une tête de forme générale triangulaire en pointe de flèche. Cette tête est une tête plate avec les deux faces 8 opposées du triangle, réalisées convergentes en direction du sommet du triangle qui forme le sommet de la tête. Chaque face 8 est une face polyédrique en forme de pointe de diamant, de dièdre ou similaire. Cette face 8 pourrait, de manière équivalente, être réalisée plane.

Les côtés 9 du triangle reliant le sommet 10 à la base 11 du triangle sont crantés à la manière de crans 12 de sapin. Les arêtes de liaison des côtés du triangle aux faces 8 du triangle sont biseautées. À nouveau, ces arêtes auraient pu être droites.

Cette forme, en pointe de flèche de la tête, permet une implantation directe de la tête dans le premier os, sans préparation préalable de l'os.

Pour parfaire l'ensemble et permettre notamment le passage d'une canule ou faciliter l'ostéogenèse, en partie centrale de l'implant, la tête 2 et la zone 4 de liaison de la tête 2 au corps 3 de l'implant sont évidées centralement. Cet évidement central forme un passage 13 axial traversant, débouchant à l'intérieur du corps 3.

L'implant 1 comprend encore, au niveau de la zone 4 de liaison de la tête 2 au corps 3, au moins deux encoches 14 aptes à permettre la saisie de l'implant 1 à l'aide d'un préhenseur 22. Chaque encoche 14 est disposée à la base d'un côté 9 du triangle. Ces encoches sont donc disposées de manière opposée, et sont obtenues grâce à un rétrécissement de la section transversale de la zone de liaison par rapport à la tête et au corps.

Dans l'espace laissé libre sur le pourtour de la pièce entre ces deux encoches, le corps peut présenter ponctuellement un élargissement qui forme, avec un flan de chaque encoche, le plus proche du corps, une surface d'appui sensiblement plane, dont le rôle sera décrit ci-après.

Un tel implant présente une longueur généralement comprise entre 10 et 30 mm. La longueur de la tête de l'implant représente au moins 20 % et au plus la moitié de la longueur totale de l'implant.

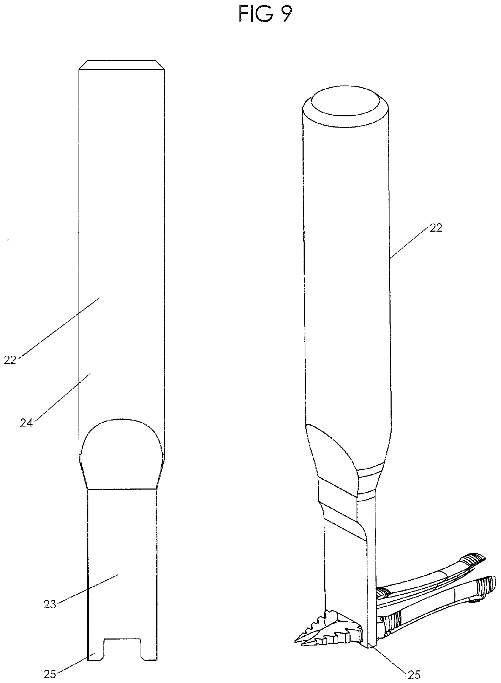

Pour la pose d'un tel implant, il est fortement recommandé d'utiliser un préhenseur 22 du type de celui représenté à la figure 9. Ce préhenseur 22 comprend un manche 24, une lame 23 disposée dans le prolongement du manche, l'extrémité de cette lame 23 étant conformée pour former deux dents 25 disposées en regard l'une de l'autre dans le plan de la lame. Chaque dent est destinée à s'insérer dans une encoche de l'implant.

Ainsi, en position de saisie de l'implant à l'aide du préhenseur, le plan de la lame du préhenseur s'étend sensiblement perpendiculairement à l'axe longitudinal de la pièce. L'implant ainsi saisi à l'aide du préhenseur peut être manipulé par le chirurgien. Celui-ci introduit le corps de l'implant dans la cavité médullaire du deuxième os, formé ici par une phalange proximale. L'insertion est facilitée par la conception des pattes longitudinales de l'implant, qui sont introduites par leur extrémité libre dans la cavité médullaire de l'os.

Cette introduction s'opère jusqu'à ce que la lame du préhenseur vienne, par l'une de ses faces dite première face, en appui sur l'extrémité du deuxième os à abouter à un os appelé premier os.

La deuxième phalange, dite distale, est emmanchée sur la tête de l'implant. Durant cet emmanchement, le préhenseur reste en place sur l'implant, de sorte que l'emmanchement s'opère jusqu'à venue de l'extrémité à abouter du premier os, en appui sur la deuxième face opposée à la première face de la lame du préhenseur.

La lame du préhenseur est ainsi prise en sandwich entre les extrémités aboutées des os, qui se trouvent ainsi éloignés l'un de l'autre d'une distance correspondant à l'épaisseur de la lame, c'est-à-dire généralement une épaisseur inférieure à deux millimètres.

À ce stade, le préhenseur peut être enlevé par un mouvement de translation orthogonal à l'axe du canal médullaire ou à l'axe longitudinal de l'implant, et les premier et deuxième os accolés l'un à l'autre par le chirurgien. Lors de cet aboutement final, le recul potentiel de l'implant à l'intérieur d'au moins l'un des os équivaut à l'épaisseur du préhenseur, soit moins de deux millimètres, ce qui n'a pas de conséquence sur la stabilité de l'implant, ce qui n'est généralement pas le cas, dans l'état de la technique, où un recul de l'implant dans le canal médullaire est observé lors de l'aboutement.

La zone de liaison de l'implant se retrouve ainsi au niveau du trait d'arthrodèse. Le préhenseur peut être pré-monté sur l'implant médical, ou livré de manière séparée.

## Revendications

1. Implant (1) médical destiné à permettre la fusion osseuse entre un premier os (20) et un deuxième os (21), ledit implant se présentant sous forme d'une pièce allongée comprenant, s'étendant suivant l'axe longitudinal de la pièce, une tête (2) et un corps (3) séparés l'un de l'autre par une zone (4) de liaison de la tête (2) au corps (3), ladite tête (2) étant apte à être insérée dans le premier os (20), ledit corps (3) étant apte à être introduit dans le deuxième os (21), ledit corps (3) étant un corps creux fendu longitudinalement sur au moins une partie de sa longueur pour délimiter au moins trois pattes (5) longitudinales avec chaque patte (5) longitudinale séparée d'une autre patte (5) longitudinale par une fente longitudinale, lesdites pattes (5) longitudinales par lesquelles ledit corps (3) est apte à être introduit dans le deuxième os (21) étant des pattes (5) élastiquement déformables,
chaque patte (5) longitudinale du corps (3) étant une patte (5) arquée qui présente une face (6) externe, c'est-à-dire tournée vers l'extérieur du corps (3), se développant longitudinalement de manière courbe avec une courbure à concavité tournée vers l'extérieur du corps (3), **caractérisé en ce que** chaque patte (5) arquée présente, dans sa partie en forme d'arc, une section transversale sensiblement constante.

2. Implant (1) médical selon la revendication 1,
**caractérisé en ce que** la tête (2) de l'implant (1) est une tête plate avec deux faces (8) opposées parallèles entre elles ou convergentes en direction du sommet de la tête (2).

3. Implant (1) médical selon l'une des revendications précédentes,
**caractérisé en ce que** la tête (2) de l'implant (1) est une tête de forme générale triangulaire en pointe de flèche.

4. Implant (1) médical selon la revendication 3,
**caractérisé en ce que** les côtés (9) du triangle reliant le sommet (10) à la base (11) du triangle sont crantés à la manière de crans (12) de sapin.

5. Implant (1) médical selon l'une des revendications précédentes,
**caractérisé en ce que** la tête (2) et la zone (4) de liaison de la tête (2) au corps (3) de l'implant (1) sont évidées centralement pour délimiter un passage (13) axial traversant débouchant à l'intérieur du corps (3).

6. Implant (1) médical selon l'une des revendications précédentes,
**caractérisé en ce que** l'implant (1) comprend, au niveau de la zone (4) de liaison de la tête (2) au corps (3), au moins deux encoches (14) aptes à permettre la saisie de l'implant (1) à l'aide d'un préhenseur (22).

7. Implant (1) médical selon la revendication 6 prise en combinaison avec l'une des revendications 3 ou 4,
**caractérisé en ce que** chaque encoche (14) est disposée à la base d'un côté (9) du triangle.

8. Implant (1) médical selon l'une des revendications précédentes,
**caractérisé en ce que** les faces (6) externes des pattes (5) sont munies au niveau des extrémités (51) libres des pattes (5) et de la zone (52) de raccordement des pattes (5) au reste du corps (3) de nervures (15) ou crans s'étendant transversalement à l'axe longitudinal des pattes (5).

9. Implant (1) médical selon l'une des revendications précédentes,
**caractérisé en ce que** les extrémités libres des pattes sont à bout arrondi.

10. Implant (1) médical selon l'une des revendications précédentes,
**caractérisé en ce que** l'arc formé, au niveau de chaque patte (5) arquée entre l'extrémité (51) libre de la patte (5) et la zone (52) de raccordement de ladite patte (5) au reste du corps (3), s'étend sur au moins 80% de la longueur de la patte (5).

11. Implant (1) médical selon l'une des revendications précédentes,
**caractérisé en ce que** chaque patte (5) arquée, dans sa partie en forme d'arc, se développe depuis l'extrémité (51) libre de la patte (5) vers sa zone (52) de raccordement au reste du corps (3), en se rapprochant de l'axe longitudinal du corps (3) puis en s'écartant de l'axe longitudinal dudit corps (3), la zone de transition entre zone de rapprochement et zone d'écartement étant disposée entre l'extrémité (51) libre de la patte (5) et les 2/3 de la longueur de la patte prise à partir de l'extrémité (51) libre de la patte (5), de préférence entre le premier tiers et le deuxième tiers de la de la longueur de la patte (5) prise à partir de l'extrémité (51) libre de la patte (5).

12. Implant (1) médical selon l'une des revendications précédentes,
**caractérisé en ce que** D1 correspondant au diamètre du cercle le plus petit à l'intérieur duquel les faces (6) externes des pattes s'inscrivent, D2 au diamètre du cercle le plus petit à l'intérieur duquel les faces (7) internes des pattes s'inscrivent et D3 à la distance minimale entre deux pattes (5) adjacentes, : D2 ≥ (D1 - D2)/2 et , de préférence, D3 ≥ (D1 - D2)/2.

13. Ensemble du type comprenant un implant (1) médical destiné à permettre la fusion osseuse entre un premier os (20) et un deuxième os (21), et un préhenseur (22),
**caractérisé en ce que** l'implant (1) médical est conforme à l'une des revendications 1 à 12.

## Patentansprüche

1. Medizinisches Implantat (1), das dazu bestimmt ist, die Knochenverschmelzung zwischen einem ersten Knochen (20) und einem zweiten Knochen (21) zu gestatten, wobei das Implantat in Form eines länglichen Teils vorliegt, umfassend, sich in der Längsachse des Teils erstreckend, einen Kopf (2) und einen Körper (3), die durch eine Verbindungszone (4) des Kopfs (2) mit dem Körper (3) voneinander getrennt sind, wobei der Kopf (2) imstande ist, in den ersten Knochen (20) eingeführt zu sein, wobei der Körper (3) imstande ist, in den zweiten Knochen (21) eingeführt zu sein, wobei der Körper (3) ein über mindestens einen Teil seiner Länge längs geschlitzter Körper ist, um mindestens drei längliche Beine (5) mit jedem länglichen Bein (5) getrennt von einem anderen länglichen Bein (5) durch einen länglichen Schlitz zu begrenzen, wobei die länglichen Beine (5), mittels derer der Körper (3) imstande ist, in den zweiten Knochen (21) eingeführt zu sein, elastisch verformbare Beine (5) sind,
wobei jedes längliche Bein (5) des Körpers (3) ein gebogenes Bein (5) ist, welches eine Außenfläche (6) aufweist, das heißt, die nach außerhalb des Körpers (3) zeigt, sich längs gekrümmt entwickelnd mit einer nach außerhalb des Körpers (3) zeigenden Konkavität,
**dadurch gekennzeichnet, dass** jedes gebogene Bein (5) in seinem bogenförmigen Teil einen etwa konstanten Querschnitt aufweist.

2. Medizinisches Implantat (1) nach Anspruch 1,
**dadurch gekennzeichnet, dass** der Kopf (2) des Implantats (1) ein flacher Kopf mit zwei zueinander parallelen oder in Richtung des Kopfs (2) konvergenten gegenüberliegenden Flächen (8) ist.

3. Medizinisches Implantat (1) nach einem der vorangehenden Ansprüche,
**dadurch gekennzeichnet, dass** der Kopf (2) des Implantats (1) ein allgemein dreieckig geformter Kopf mit pfeilförmiger Spitze ist.

4. Medizinisches Implantat (1) nach Anspruch 3,
**dadurch gekennzeichnet, dass** die Seiten (9) des Dreiecks, welche die Spitze (10) mit der Basis (11) des Dreiecks verbinden, in der Art von Zacken (12) einer Tanne gezackt sind.

5. Medizinisches Implantat (1) nach einem der vorangehenden Ansprüche,
**dadurch gekennzeichnet, dass** der Kopf (2) und die Verbindungszone (4) des Kopfs (2) mit dem Körper (3) des Implantats (1) zentral ausgehöhlt sind, um einen axialen durchgängigen Durchgang (13) zu bilden, der im Inneren des Körpers (3) ausmündet.

6. Medizinisches Implantat (1) nach einem der vorangehenden Ansprüche,
**dadurch gekennzeichnet, dass** das Implantat (1) im Bereich der Verbindungszone (4) des Kopfs (2) mit dem Körper (3) mindestens zwei Kerben (14) umfasst, die imstande sind, das Ergreifen des Implantats (1) mit Hilfe eines Greifers (22) zu gestatten.

7. Medizinisches Implantat (1) nach Anspruch 6, herangezogen in Kombination mit einem der Ansprüche 3 oder 4,
**dadurch gekennzeichnet, dass** jede Kerbe (14) an der Basis einer Seite (9) des Dreiecks angeordnet ist.

8. Medizinisches Implantat (1) nach einem der vorangehenden Ansprüche,
**dadurch gekennzeichnet, dass** die Außenflächen (6) der Beine (5) im Bereich der freien Enden (51) der Beine (5) und der Verbindungszone (52) der Beine (5) mit dem Rest des Körpers (3) mit Rippen (15) oder Zacken versehen sind, die sich quer zur Längsachse der Beine (5) erstrecken.

9. Medizinisches Implantat (1) nach einem der vorangehenden Ansprüche,
**dadurch gekennzeichnet, dass** die freien Enden der Beine abgerundet sind.

10. Medizinisches Implantat (1) nach einem der vorangehenden Ansprüche,
**dadurch gekennzeichnet, dass** sich der im Bereich jedes gebogenen Beins (5) zwischen dem freien Ende (51) des Beins (5) und der Verbindungszone (52) des Beins (5) mit dem Rest des Körpers (3) gebildete Bogen über mindestens 80 % der Länge des Beins (5) erstreckt.

11. Medizinisches Implantat (1) nach einem der vorangehenden Ansprüche,
**dadurch gekennzeichnet, dass** sich jedes gebogene Bein (5) in seinem bogenförmigen Teil ab dem freien Ende (51) des Beins (5) in Richtung seiner Verbindungszone (52) mit dem Rest des Körpers (3) entwickelt, indem es sich an die Längsachse des Körpers (3) annähert, sich dann von der Längsachse des Körpers (3) beabstandet, wobei die Übergangszone zwischen Annäherungszone und Beabstandungszone zwischen dem freien Ende (51) des Beins (5) und den 2/3 der Länge des Beins, herangezogen ab dem freien Ende (51) des Beins (5), vorzugsweise zwischen dem ersten Drittel und dem zweiten Drittel der Länge des Beins (5), herangezogen ab dem freien Ende (51) des Beins (5), angeordnet ist.

12. Medizinisches Implantat (1) nach einem der vorangehenden Ansprüche,
**dadurch gekennzeichnet, dass** D1 dem Durchmesser des kleinsten Kreises, in dessen Inneren die Außenflächen (6) der Beine verlaufen, D2 dem Durchmesser des kleinsten Kreises, in dessen Inneren die Innenflächen (7) der Beine verlaufen, und D3 dem Mindestabstand zwischen zwei benachbarten Beinen (5) entspricht,
: D2 ≥ (D1 - D2)/2 und vorzugsweise D3 ≥ (D1 - D2)/2.

13. Einheit des Typs, umfassend ein medizinisches Implantat (1), das dazu bestimmt ist, die Knochenverschmelzung zwischen einem ersten Knochen (20) und einem zweiten Knochen (21) zu gestatten, und einen Greifer (22),
**dadurch gekennzeichnet, dass** das medizinische Implantat (1) nach einem der Ansprüche 1 bis 12 ist.

## Claims

1. A medical implant (1) suitable for allowing bone fusion between a first bone (20) and a second bone (21), said implant assuming the form of an elongate part comprising, extending along the longitudinal axis of the part, a head (2) and a body (3) that are separated from one another by a zone (4) for connecting the head (2) to the body (3), said head (2) being capable of being inserted into the first bone (20), said body (3) being capable of being introduced into the second bone (21), said body (3) being a hollow body with a longitudinal slit over at least part of its length to delimit at least three longitudinal tabs (5) with each longitudinal tab (5) separated from another longitudinal tab (5) by a longitudinal slit, said longitudinal tabs (5) by which said body (3) is capable of being introduced into said second bone (21) being resiliently deformable tabs (5), each longitudinal tab (5) of the body (3) being a bowed tab (5) that has an outer face (6), that is to say, facing toward the outside of the body (3), developing longitudinally in a curved manner with a curvature having its concave side turned toward the outside of the body (3), **characterized in that** each bowed tab (5) has, in its arc-forming part, a substantially constant cross-section.

2. The medical implant (1) according to claim 1,
**characterized in that** the head (2) of the implant (1) is a flat head with two opposite faces (8) that are parallel to one another or that converge toward the apex of the head (2).

3. The medical implant (1) according to one of the preceding claims,
**characterized in that** the head (2) of the implant (1) is a generally triangular, arrowhead-shaped head.

4. The medical implant (1) according to claim 3,
**characterized in that** the sides (9) of the triangle connecting the apex (10) to the base (11) of the triangle are indented like the indentations (12) in a Christmas tree shape.

5. The medical implant (1) according to one of the preceding claims,
**characterized in that** the head (2) and the area (4) for connecting the head (2) to the body (3) of the implant (1) are hollowed out centrally in order to delimit an axial through passage (13) emerging inside the body (3).

6. The medical implant (1) according to one of the preceding claims,
**characterized in that** the implant (1) comprises, at the area (4) for connecting the head (2) to the body (3), at least two notches (14) capable of allowing the implant (1) to be grasped using a gripper (22).

7. The medical implant (1) according to claim 6 in combination with one of claims 3 or 4,
**characterized in that** each notch (14) is arranged at the base of one side (9) of the triangle.

8. The medical implant (1) according to one of the preceding claims,
**characterized in that** the outer faces (6) of the tabs (5) are provided, at the free end (51) of the tabs (5) and the area (52) for connecting the tabs (5) to the rest of the body (3), with ribs (15) or indentations extending transversely to the longitudinal axis of the tabs (5).

9. The medical implant (1) according to one of the preceding claims,
**characterized in that** the free ends of the tabs have a rounded end.

10. The medical implant (1) according to one of the preceding claims,
**characterized in that** the arc formed, at each bowed tab (5) between the free end (51) of the tab (5) and the area (52) for connecting said tab (5) to the rest of the body (3), extends over at least 80% of the length of the tab (5).

11. The medical implant (1) according to one of the preceding claims,
**characterized in that** each bowed tab (5), in its arc-forming part, develops from the free end (51) of the tab (5) toward its area (52) for connecting to the rest of the body (3), while moving closer to the longitudinal axis of the body (3) then moving away from the longitudinal axis of said body (3), the transitional area between the area in which it moves closer and the area in which it moves further away being arranged between the free end (51) of the tab (5) and 2/3 of the length of the tab taken from the free end (51) of the tab (5), preferably between the first third and the second third of the length of the tab (5) taken from the free end (51) of the tab (5).

12. The medical implant (1) according to one of the preceding claims,
**characterized in that** D1 corresponding to the diameter of the smallest circle inside which the outer faces (6) of the tabs fit, D2 to the diameter of the smallest circle inside which the inner faces (7) of the tabs fit and D3 to the minimum distance between two adjacent tabs (5): D2 ≥ (D1 - D2)/2, and preferably, D3 ≥ (D1 - D2)/2.

13. A kit of the type comprising a medical implant (1) intended to allow the bone fusion between a first bone (20) and a second bone (21), and a gripper (22),
**characterized in that** the medical implant (1) is according to one of claims 1 to 12.
